# EUROPEAN PATENT APPLICATION

(11) **EP 4 756 015 A1**
(43) Date of publication of application: **10.06.2026**
(21) Application number: 24847664.0
(22) Date of filing: 22.04.2024
(51) Int. Cl.: C12N 9/02, C12N 15/53, C12P 17/02

(54) **BIOLOGICAL ENZYME FOR CATALYZING HYDROXYLATION OF C9 SITE IN TAXANE COMPOUND, NUCLEIC ACID MOLECULE, BIOLOGICAL MATERIAL AND USE OF BIOLOGICAL ENZYME, NUCLEIC ACID MOLECULE AND BIOLOGICAL MATERIAL**

(30) Priority: 31.07.2023 CN 202310961179
(71) Applicant: Agricultural Genomics Institute at Shenzhen, Chinese Academy of Agricultural Sciences, Shenzhen, Guangdong 518124 (CN); Agricultural Genomics Institute, Chinese Academy of Agricultural Sciences, Shenzhen, Guangdong 518124 (CN)
(72) Inventor: YAN, Jianbin, Shenzhen, Guangdong 518124 (CN)
(74) Representative: Murgitroyd & Company
(86) International application number: PCT/CN2024/089045
(87) International publication number: WO 2025/025698

(57) **Abstract**

The disclosure relates to the technical field of plant metabolic biology and synthetic biology, in particular to a bioenzyme for catalyzing hydroxylation of C9 position in a taxane compound, and a nucleic acid molecule, a biological material and use thereof. The bioenzyme includes: at least one of the amino acid sequences represented by SEQ ID NOs: 1-14; and/or a fused amino acid sequence thereof; and/or an amino acid sequence obtained by substitution and/or deletion and/or addition of one or more amino acid residues and having the same function; and/or an amino acid sequence having at least 60% identity and having the same function. The successful analysis of the bioenzyme is beneficial to realize the total biosynthesis of paclitaxel and improve the biosynthesis yield of baccatin III, thereby solving the problem of short supply of paclitaxel.

## Description

### TECHNICAL FIELD

The present disclosure relates to the technical field of plant metabolic biology and synthetic biology, and particularly to a bioenzyme for catalyzing hydroxylation of C9 position in a taxane compound, and a nucleic acid molecule, a biological material and use thereof.

### BACKGROUND

Taxanes are anti-tumor active ingredients isolated from plants, and the structures of resulting active ingredient compounds are modified to synthesize a series of derivatives. They are important first-line anti-tumor drugs. Taxane drugs mainly include paclitaxel (a compound represented by the formula III below), docetaxel, cabazitaxel, and derivatives having a taxane skeleton structure. Currently, the main production method for obtaining paclitaxel raw material ingredients is the semi-synthetic method, which is firstly extracting the natural precursors baccatin III and 10-deacetylbaccatin III (10-DAB), and then chemically synthesizing paclitaxel. However, the precursor substances used in this method still rely on extraction from the bark or leaf cells of *Taxus chinensis.* Due to the scarce resources of *Taxus chinensis,* the supply problem cannot be completely solved.

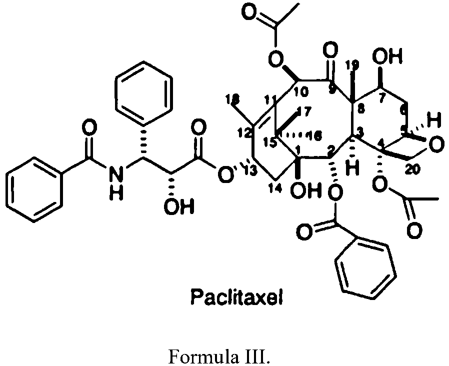

The reason why baccatin III and 10-deacetyl baccatin III can only be extracted from scarce *Taxus chinensis* is that: several key enzymes in their synthesis pathway still have not been elucidated, one of which is the enzyme for catalyzing the oxidation reaction at the C9 position. Since baccatin III includes 7 single oxidation sites, and the oxidation order and oxidation substrate are unknown, it is extremely difficult to screen an enzyme for catalyzing the C9 oxidation reaction (i.e., C9 hydroxylase, T9αH), as a result, this enzyme is not being identified in the past three decades.

Based on the above situation, there is an urgent need to comprehensively analyze the key enzymes in the synthesis of paclitaxel, especially the key enzymes in the synthesis of natural precursors baccatin III and 10-deacetylbaccatin III, such as C9 hydroxylase, and then utilize synthetic biology and metabolic engineering strategies to reconstruct the paclitaxel synthesis pathway in plants or microbial chassis, so as to mass produce paclitaxel and completely solve the problem of paclitaxel supply and demand.

### SUMMARY

In view of this, the present disclosure provides a bioenzyme for catalyzing hydroxylation of C9 position in a taxane compound, and a nucleic acid molecule, a biological material and use thereof. The successful analysis of this bioenzyme (also known as C9 hydroxylase, T9αH) will help to achieve the total biosynthesis of paclitaxel, and increase the yield of baccatin III biosynthesis, thereby solving the problem of paclitaxel supply and demand.

In order to achieve the above object of the disclosure, the present disclosure provides the following technical solutions.

The present disclosure provides a bioenzyme T9αH, where its amino acid sequence includes at least one of the following sequences:
a1) at least one of amino acid sequences represented by SEQ ID NOs: 1-14;
a2) a fusion amino acid sequence obtained by connecting a tag to N-terminus and/or C-terminus of at least one of the amino acid sequences represented by SEQ ID NOs: 1-14;
a3) an amino acid sequence obtained by substitution and/or deletion and/or addition of one or more amino acid residues in at least one of the amino acid sequences represented by SEQ ID NOs: 1-14 and having the same function; or
a4) an amino acid sequence having at least 60% identity with at least one of the amino acid sequences represented by SEQ ID NOs: 1-14 and having the same function.

Optionally, the amino acid sequence of a4) is derived from the genus *Taxus,* including but not limited to: *Taxus baccata L., Taxus brevifolia* Nutt., *Taxus calcicola* L.M. Gao & Mich. Möller, *Taxus canadensis* Marshall, *Taxus chinensis* (Pilg.) Rehd., *Taxus contorta* Griff., *Taxus cuspidata* Siebold & Zucc., *Taxus Florida* Nutt ex Chapm., *Taxus florini* Spjut, *Taxus globosa* Schltdl., *Taxus mairei* (Lemée & Lév.) S.Y. Hu, *Taxus phytoni* Spjut, *Taxus sumatrana* (Miq.) de Laub, *Taxus wallichiana* Zucc., *Taxus* Huangshan type, *Taxus* Emei type, *Taxus* Qinling type, *Taxus yunnanensis W.* C. Cheng&L. K. Fu, *Taxus* × *media* Rehder, *Taxus* × *hunnewelliana* Rehder, and others.

The present disclosure creatively identifies a bioenzyme from *Taxus chinensis* which can catalyze the hydroxylation reaction at the C9 position in the synthesis of taxane compounds, and the present disclosure names it T9αH. It is found in functional analysis that T9αH can catalyze hydroxylation of C9 position in compounds with a paclitaxel carbon skeleton structure and a non-hydroxylated C9 position, thereby opening up a biosynthetic pathway of taxane intermediates such as baccatin III or a precursor thereof, and further realizing the total biosynthesis of taxanes such as paclitaxel.

In a particular embodiment of the present disclosure, when synthesizing taxusin, T9αH enzyme can catalyze the conversion of taxadiene-5α,10β,13α-triol into taxadiene-5α,9α,10β,13α-tetraol to achieve a hydroxylation reaction at the C9 position.

In another particular embodiment of the present disclosure, when synthesizing baccatin III (paclitaxel precursor), T9αH enzyme can catalyze hydroxylation of C9 position in a compound with a paclitaxel carbon skeleton structure and a non-hydroxylated C9 position, thereby successfully synthesizing baccatin III.

Furthermore, the present disclosure obtains homologous proteins and mutants of T9αH, proving that the homologous proteins and mutants can also catalyze hydroxylation of C9 position.

In a particular embodiment of this disclosure, in the fusion proteins obtained by connecting a tag to N-terminus and/or C-terminus of the amino acid sequences represented by SEQ ID NOs: 1-14. The connecting tag is not particularly limited and can be added according to the purpose of use. For example, it may be selected from, but is not limited to: HIS GST, Flag, MBP, HA, c-Myc, eGFP, eYFP, and eCFP.

In an embodiment of the present disclosure, the amino acid sequence of a3) is an amino acid sequence obtained by substitution and/or deletion and/or addition of one or more (e.g., 1, 2, 3, 4, 5, 6, 7, 8, 9, 10 or more) amino acid residues in the amino acid sequence of a1) and having the same bioenzyme activity function.

In an embodiment of the present disclosure, the amino acid sequence of a3) is an amino acid sequence obtained by substitution and/or deletion and/or addition of one or more (e.g., 1, 2, 3, 4, 5, 6, 7, 8, 9, 10 or more) amino acid residues in the amino acid sequence of a1) and having similar or better bioenzyme activity function.

In an embodiment of the present disclosure, the amino acid sequence of a4) is an amino acid sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity with the amino acid sequence of a1) and having the same bioenzyme activity function.

In an embodiment of the present disclosure, the amino acid sequence of a4) is an amino acid sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity with the amino acid sequence of a1) and having similar or better bioenzyme activity function.

In particular embodiments of the present disclosure, the sequence similarity of the above 14 T9αH amino acid sequences is 63.15%-100%, and the conserved domains/sites are the conserved PSRF motif (amino acids 423-426), the highly conserved heme-binding PFG element (amino acids 439-441), the ETMR salt bridge (amino acids 369-372), the EXXR motif and the key cysteine (amino acid 447); the key amino acid sites of the mutants include L75, G128, P129, F132, V136, V142, L228, T231, L232, F305, H308, A309, D312, T313, S316, P376, A377, F378, G379, S380, F381, R382, M402, F485, P486, and P487. The above amino acids are represented by single-letter abbreviations. The amino acids, English names and English abbreviations are shown in Table 4.

In a particular embodiment of the present disclosure, the above bioenzyme is an isolated bioenzyme.

In a particular embodiment of this disclosure, the homologous sequence of the amino acid sequence represented by SEQ ID NO: 1 is the amino acid sequence represented by SEQ ID NOs: 2-14. The homology between the homologous sequences represented by SEQ ID NOs: 2-14 and the amino acid sequence represented by SEQ ID NO: 1 is 63.15-100%.

The disclosure further provides a nucleic acid molecule encoding the above bioenzyme, the nucleotide sequence of which include at least one of the following sequences:
b1) at least one of the nucleotide sequences represented by SEQ ID NOs: 15-28;
b2) a complementary sequence, a degenerate sequence or a homologous sequence of at least one of the nucleotide sequences represented by SEQ ID NOs: 15-28, where the homologous sequence is a nucleotide sequence having at least 75% identity with the nucleotide sequence represented by SEQ ID NOs: 15-28; or
b3) a nucleotide sequence hybridizing with the nucleotide sequence of b1) or b2) under stringent conditions and being capable of encoding a protein with the same function.

In an embodiment of the disclosure, the complementary sequence is a complementary sequence formed according to the principle of complementary base pairing, and the complementary sequence may be an incomplete complementary sequence or a complete complementary sequence having the same function as the nucleotide sequence of b1).

In the embodiment of the disclosure, a degenerate sequence means that after changing one or more nucleotides in the nucleotide sequence of b1), the type of amino acid encoded by the changed nucleotide sequence position remains unchanged, and the function and expression level of the nucleic acid molecule will not be affected.

In an embodiment of the disclosure, a homologous sequence is a nucleotide sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity with the nucleotide sequence of b1) and having the same function; for example, a mutant gene, an allele, or a derivative.

Optionally, the nucleotide sequence of the nucleic acid molecule of b2) is derived from the genus *Taxus,* including but not limited to: *Taxus baccata L., Taxus brevifolia* Nutt., *Taxus calcicola* L.M. Gao & Mich. Möller, *Taxus canadensis* Marshall, *Taxus chinensis* (Pilg.) Rehd., *Taxus contorta* Griff., *Taxus cuspidata* Siebold & Zucc., *Taxus Florida* Nutt ex Chapm., *Taxus florini* Spjut, *Taxus globosa* Schltdl., *Taxus mairei* (Lemée&Lév.) S.Y. Hu, *Taxus phytoni* Spjut, *Taxus sumatrana* (Miq.) de Laub, *Taxus wallichiana* Zucc., *Taxus* Huangshan type, *Taxus* Emei type, *Taxus* Qinling type, *Taxus yunnanensis* W. C. Cheng&L. K. Fu, *Taxus* × *media* Rehder, *Taxus* × *hunnewelliana* Rehder, and others. Optionally, the nucleic acid molecule is a synthetic and/or isolated nucleic acid molecule.

Illustratively, "stringent conditions" refer to conditions under which a probe will hybridize to its target sequence to a detectable degree exceeding hybridization to other sequences (e.g., at least 2-fold over background). Stringent conditions are sequence-dependent, and will be different in different circumstances. By controlling the stringency of the hybridization and/or washing conditions, target sequences that are 100% complementary to a probe can be identified. Alternatively, stringency conditions can be adjusted to allow some sequence mismatching so that lower degrees of similarity are detected.

In a particular embodiment of the present disclosure, the sequence similarity of the nucleotide sequence encoding T9αH is 75.48-100%.

In a particular embodiment of the present disclosure, the homologous sequence of the nucleotide sequence represented by SEQ ID NO: 15 is the nucleotide sequences represented by SEQ ID NOs: 16-28. The homology between the homologous sequences represented by SEQ ID NOs: 16-28 and the nucleotide sequence represented by SEQ ID NO: 15 is 75.48-100%.

The present invention also provides a bioenzyme composition including the above bioenzyme T9αH, as well as at least one of: TXS, TAT, T5αH, T13αH, T2αH, T7βH, TOT, or TBT.

Preferably, the bioenzyme composition includes all of the above bioenzymes.

The present disclosure provides a biomaterial, which is any one of the following c1) to c3):
c1) an expression cassette including the above nucleic acid molecule;
c2) a vector including the above nucleic acid molecule or the expression cassette of c1); and
c3) a host cell including at least one of: the above nucleic acid molecule, the expression cassette of c1) and the vector of c2).

Optionally, the host cell includes at least one of: microbial cell, plant cell, animal cell, or algae cell.

In an embodiment of the disclosure, the expression cassette of c1) further includes a promoter operatively connected to the above nucleic acid molecule.

Optionally, the expression cassette of c1) further includes other regulatory elements, such as enhancer, leader sequence, transposon, terminator, marker gene, etc.

In the embodiment of the disclosure, in the recombinant vector of c2), the type of the vector is not particularly limited, and a suitable vector can be selected according to practical needs. For example, the vectors include but are not limited to: pFastBac1, pYES2, pYES2.1, pESC-Ura, pESC-Trp, pESC-Leu, pESC-His, pGEX2T, pTAex3, pUSA, pYMB0, pHT43, pET28b, pIJ702, pUCP19, pEAQ-HT, pYMB03 or pHT43; preferably pEAQ-HT.

In an embodiment of the disclosure, in the host cell of c3), the microbial cell includes but is not limited to at least one of: *Streptomyces, Pseudomonas, Bacillus,* yeast cell, or *Escherichia coli.*

In the embodiments of the disclosure, in the host cell of c3), the microbial cell includes bacteria and/or fungi.

In an embodiment of the disclosure, the bacteria include but are not limited to: *Escherichia* cells, *Lactobacillus* cells, *Lactococcus* cells, *Corynebacterium* bacteria, *Acetobacter* bacteria, *Acinetobacter* bacteria, *Pseudomonas* cells, *Streptomyces* cells, *Bacillus* cells, *Staphylococcus* cells, *Agrobacterium* cells, and endophytes of *Taxus.* For example, the bacteria include but are not limited to: *Escherichia coli, Bacillus subtilis, Agrobacterium tumefaciens, Agrobacterium rhizogenes, Lactococcus lactis, Bacillus cereus, Pseudomonas fluorescens,* etc. In some embodiments, bacterial cells include but are not limited to: such as *Escherichia* spp., *Streptomyces* spp., *Zymonas* spp., *Acetobacter* spp., *Citrobacter* spp., *Synechocystis* spp., *Rhizobium* spp., *Clostridium* spp., *Corynebacterium* spp., *Streptococcus* spp., *Xanthomonas* spp., *Lactobacillus* spp., *Lactococcus* spp., *Bacillus* spp., *Alcaligenes* spp., *Pseudomonas* spp., *Aeromonas* spp., *Azotobacter* spp., *Comamonas* spp., *Mycobacterium* spp., *Rhodococcus* spp., *Gluconobacter* spp., *Ralstonia* spp., *Acidithiobacillus* spp., *Microlunatus* spp., *Geobacter* spp., *Geobacillus* spp., *Arthrobacter* spp., *Flavobacterium* spp., *Serratia* spp., *Saccharopolyspora* spp., *Thermus* spp., *Stenotrophomonas* spp., *Chromobacterium* spp., *Sinorhizobium* spp., *Saccharopolyspora* spp., *Agrobacterium* spp., *and Pantoea* spp.. The bacterial cells may be Gram-negative cells, such as *E. coli* cells, or Gram-positive cells, such as *Bacillus* species.

In an embodiment of the disclosure, the fungi include but are not limited to: yeast, filamentous fungus or mushroom.

In a particular embodiment of the disclosure, yeast includes but is not limited to: *Saccharomyces cerevisiae, Schizosaccharomyces pombe, Yarrowia lipolytica, Candida utilis, Pichia pastoris, Kluyveromyces lactis, Hansenula polymorpha, Candida boidinii, Adenysera adenilyticus, Phaffia rhodozyma, and Candida albicans.* The yeast cells are, for example, *Saccharomyces* spp., *Schizosaccharomyces* spp., *Pichia* spp., *Paffia* spp., *Kluyveromyces* spp., *Candida* spp., *Talaromyces* spp., *Brettanomyces* spp., *Pachysolen* spp., *Debaryomyces* spp., *Yarrowia* spp. and industrial polyploid yeast strains. Preferably, the yeast strain is a S. *cerevisiae* strain or a *Yarrowia* strain.

In a particular embodiment of the disclosure, the filamentous fungus includes but is not limited to: *Monascus, Aspergillus oryzae, Aspergillus niger, Aspergillus flavus, and Penicillium.* The fungus is, for example, *Aspergillus* spp., *Penicillium* spp., *Fusarium* spp., *Rhizopus* spp., *Acremonium* spp., *Neurospora* spp., *Sordaria* spp., *Magnaporthe* spp., *Allomyces* spp., *Ustilago* spp., *Botrytis* spp. and *Trichoderma* spp..

In an embodiment of the present disclosure, in the host cell of c3), the plant cell includes but is not limited to at least one of: tobacco cell, *Pseudotaxus chienii* cell, *Artemisia annua* cell, *Arabidopsis* cell, *Physcomitrella patens* cell, *Marchantia polyphylla* cell, tomato cell, ginseng cell, cotton cell, sugarcane cell, potato cell, corn cell, wheat cell, rice cell, radish cell, lettuce cell, etc., and the plant cell include protoplasts, suspension cell, etc.

In an embodiment of the present disclosure, in the host cell of c3), the animal cell includes but is not limited to at least one of: insect cell, mammalian cell, fruit fly cell, nematode cell, or fish cell.

In a particular embodiment of the disclosure, insect cell includes but is not limited to: S2 *Drosophila* cells or Sf9 cell.

In a particular embodiment of the disclosure, mammalian cell includes but is not limited to: fibroblast, lymphocyte, epithelial cell, and myeloblast. For example, mammalian cell includes but is not limited to: HEK293 cell, CHO cell, COS cell, BHK cell, HeLa cell, Chinese hamster ovary cell, Vero cell, SP2/0 cell, NS/0 myeloma cell, hamster kidney cell, human B cell, human T cell Jurkat, neuronal cell, CV-I/EBNA cell, L cell, 3T3 cell, HEPG2 cell, and MDCK cell.

In a particular embodiment of the disclosure, fish cell includes but is not limited to zebrafish cell.

In an embodiment of the present disclosure, the host cell of c3) includes but is not limited to at least one of: cyanobacteria, green algae, *Synechococcus,* elongated *Synechococcus, Synechocystis, Anabaena, Chlamydomonas,* or *Chlamydomonas reinhardtii.*

In an embodiment of the present disclosure, the host cell further includes at least one of TXS, TAT, T5 alpha H, T13 alpha H, T2 alpha H, T7 beta H, TOT, or TBT.

The present disclosure also provides an engineered strain, which includes at least one of the above nucleic acid molecules, expression cassettes or vectors. In an embodiment of the present disclosure, the engineered strain may be a yeast engineered strain, or an *Escherichia coli* engineered strain.

In an embodiment of the present disclosure, the plant cells and/or animal cells are non-biologically transformed recombinant plant cells and/or recombinant animal cells.

In an embodiment of the present disclosure, optionally, the plant cells and/or animal cells are non-biologically transformed (produced by a non-substantial biological method) recombinant plant cells and/or recombinant animal cells.

Optionally, plant cells or animal cells can express bioenzymes and produce target products through cell culture.

Among the above biological materials, methods for transforming host cells with nucleic acid molecules, expression cassettes, and vectors in vivo and in vitro include but are not limited to: electroporation, polyethylene glycol (PEG) transformation, lipofection, heat shock, calcium phosphate precipitation, virus-mediated, and microinjection.

The disclosure further provides a method for producting a host cell, where the method includes transforming the host cell with at least one of the above nucleic acid molecules, expression cassettes or vectors in biological materials.

In the embodiments of the present disclosure, the method for producting host cells further includes transforming host cells with at least one of: TXS, TAT, T5αH, T13αH, T2αH, T7βH, TOT, or TBT.

In the embodiments of the disclosure, the host cell includes microbial cell, plant cell, animal cell, and/or algal cell.

The disclosure further provides a method for producing producting a plants or plant cell, the method includes transforming the plant or plant cell with at least one of the above nucleic acid molecules, expression cassettes or vectors in biological materials.

In the embodiments of the present disclosure, the method for producting plants or plant cells further includes transforming the plants or plant cells with at least one of: TXS, TAT, T5αH, T13αH, T2αH, T7βH, TOT, or TBT.

This disclosure provides a method for producting the above bioenzyme, where the method includes: transforming a host cell with at least one of the above nucleic acid molecules, or the expression cassette or vector in the biological material, so that the host cell produces the bioenzyme.
In the embodiments of the disclosure, the host cell includes microbial cell, plant cell, animal cell, and/or algal cell.

The present disclosure provides use of any one of the above bioenzymes, nucleic acid molecules or biological materials in the following d1), d2), d3) and/or d4):
d1) catalyzing hydroxylation of C9 position in a precursor substance of a taxane compound and/or an intermediate thereof; the precursor substance of the taxane compound and/or an intermediate thereof is a precursor substance in which the C9 position has not been hydroxylated or oxidized;
d2) synthesizing taxusin and/or an intermediate thereof;
d3) synthesizing baccatin III and/or an intermediate thereof; and
d4) synthesizing a taxane compound and/or an intermediate thereof.

In an embodiment of the present disclosure, the taxane compound includes but is not limited to at least one of paclitaxel and a derivative thereof, taxusin and a derivative thereof, docetaxel and a derivative thereof, or cabazitaxel and a derivative thereof.

In an embodiment of the present disclosure, the taxane compound intermediate includes but is not limited to at least one of: taxadiene-hexaol-hexaacetate and a derivative thereof, 1-dehydroxybaccatin IV and a derivative thereof, baccatin III and a derivative thereof, 10-deacetylbaccatin III and a derivative thereof, paclitaxel and a derivative thereof, baccatin IV and a derivative thereof, baccatin VI and a derivative thereof, baccatin VII and a derivative thereof, baccatin IX and a derivative thereof, baccatin V and a derivative thereof, taxusin and a derivative thereof, baccatin I and a derivative thereof, taxadiene-2α,5α,7β,9β,10β,13α-hexaol-5α-acetate and a derivative thereof, 5α-acetate-taxuspine F and a derivative thereof, taxadiene-hexaol-tetraacetate and a derivative thereof, or taxuspine F and a derivative thereof.

In an embodiment of the present disclosure, the disclosure includes the following aspects.
(1) After removing or substituting certain amino acids, the amino acid sequence of the bioenzyme provided by the present disclosure or at least a part of its polypeptide sequence may still have biological activity or even new biological activity, or improve the yield or optimize the protein kinetic characteristics or other properties to be obtained.
(2) The nucleotide sequence of the nucleic acid molecule provided by the present disclosure or at least a part of its nucleotide sequence is modified or mutated, where the modification or mutation methods include: codon optimization, insertion, deletion, polymerase chain reaction (PCR), error-prone PCR, reconnection of different sequences, directed evolution of different parts of the sequence or its homologous sequences from other sources, or mutagenesis by chemical reagents, etc.
(3) The cloned gene of nucleotide sequence provided by the present disclosure or at least a part of its nucleotide sequence is expressed in an exogenous host through a suitable expression system, so as to obtain the corresponding enzyme or other higher biological activity or yield.
(4) The above polynucleotide or protein is overexpressed in a recipient host cell to obtain a target host cell, where the target host cell has an increased production of paclitaxel and/or an intermediate thereof as compared to the recipient host. Methods for overexpressing the above polynucleotide or protein in a recipient host include one or more of: promoter editing technology, codon optimization, use of a strong promoter, insertion of an intron, use of a viral vector, fusion protein technology, and other methods that can achieve the purpose of overexpression.
(5) The gene or gene cluster of the nucleotide sequence or at least a part of its nucleotide sequence provided by the present disclosure can be used to construct a recombinant plasmid through genetic recombination, so as to obtain a new biosynthetic pathway, or to obtain a new biosynthetic pathway through insertion, substitution, deletion or inactivation.

The present disclosure provides a method for synthesizing a taxane compound and/or an intermediate thereof in vivo or in vitro, where the method includes utilizing at least one of the precursor substance of a taxane compound and/or an intermediate thereof as a substrate to hydroxylate the C9 position under the catalytic action of the above bioenzyme; and where the precursor substance of the taxane compound and/or an intermediate thereof is a precursor substance in which the C9 position has not been hydroxylated and oxidized.

In an embodiment of the present disclosure, the precursor substance of the taxane compound and/or an intermediate thereof includes a compound represented by Formula I and/or Formula II: 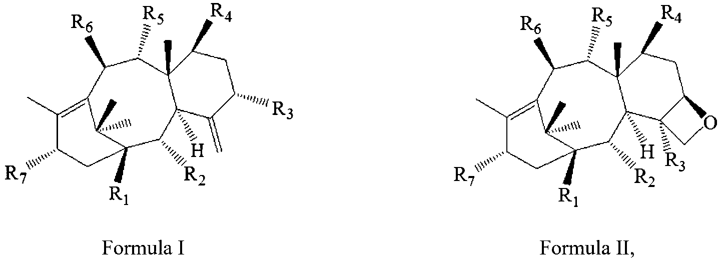 where R₁, R₃ and R₄ are each independently selected from: H, -OH, and -OAc (acetoxyl, -OOCCH₃); R₂ and R₆ are each independently selected from: H, -OH, -OAc, and -Obz (benzoyl, C₆H₃-CO-CH₂-); R₅ and R₇ are each independently selected from: H, -OH, -OAc, and =O.

In a particular embodiment of the present disclosure, the precursor substance of the taxane compound and/or an intermediate thereof includes but is not limited to at least one of: taxa-4(5),11(12)-diene, taxa-4(20),11(12)-diene-5α-ol, taxadiene-5α,10β,13α-triol, and/or a derivative thereof.

In an embodiment of the present disclosure, the bioenzyme for synthesizing a taxane compound and/or an intermediate thereof further includes at least one of: TXS, TAT, T5αH, T13αH, T2αH, T7βH, TOT, TBT, T10βH, T14βH, DBAT, BAPT, T2'OH, DBTNBT, PAM, CoAligase, TAX9, TAX14, or TAX19.

Particularly, the bioenzyme (T9αH), TXS, TAT, T5αH, T13αH, T2αH, T7βH, TOT, and TBT according to the present disclosure are the bioenzymes required for synthesizing baccatin III from geranylgeranyl pyrophosphate (GGPP); DBAT, BAPT, T2'OH, DBTNBT, PAM, and CoA ligase are the bioenzymes required for synthesizing taxane compounds such as paclitaxel from baccatin III. Enzymes for synthesizing other taxanes include T10βH, T14βH, TAX9, TAX14, and TAX19.

In an embodiment of the present disclosure, in vivo includes within a microbial cell, within an algae cell, within a plant cell, and/or within an animal cell.

In an embodiment of the present disclosure, the taxane compound and/or an intermediate thereof are synthesized in vivo, the method includes allowing the above nucleic acid molecule to express a bioenzyme in microbial cell, algal cell, plant cell, and/or animal cell; and then utilizing the precursor substance of the taxane compound and/or an intermediate thereof as a substrate to synthesize the taxane compound and/or an intermediate thereof under the catalytic action of the bioenzyme. Optionally, the substrate may be added artificially in addition.

In the particular embodiment of the present disclosure, the method for expressing the bioenzyme according to the present disclosure includes transforming a host cell with at least one of: the above the nucleic acid molecule, or the expression cassette or vector in the biological material, so that the host cell produces the bioenzyme.

This disclosure also provides a method for synthesizing baccatin III by using geranylgeranylpyrophosphate GGPP as a substrate to synthesize baccatin III, under the action of bioenzymes T9αH, TXS, TAT, T5αH, T13αH, T2αH, T7βH, TOT, and TBT.

Preferably, the method for synthesizing baccatin III includes synthesizing baccatin III by using the above host cell, where the host cell includes heterologous T9αH, TXS, TAT, T5αH, T13αH, T2αH, T7βH, TOT, and TBT.

The present disclosure provides a plant or a plant part thereof, where the plant is one of the following plants:
e1) a transgenic plant including the above nucleic acid molecule;
e2) a plant formed by growth of the above plant cell;
e3) a plant produced by the above method;
e4) an offspring formed by self-pollination of any one of the plants of e1) to e3), as well as a plant formed by growth of the offspring; and
e5) an offspring formed by hybridization of any one of the plants of e1) to e3) with other varieties, as well as a plant formed by growth of the offspring;
where the above plant part includes a root, stem, leaf, flower, fruit, pollen or seed.

The disclosure further provides a method for manufacturing a commercial product, the method includes: obtaining the above plant or plant part thereof, and manufacturing the commercial product from the plant or plant part thereof; where the commercial product is a crude extract, an active pharmaceutical ingredient, and/or a pharmaceutical formulation including at least one selected from the group consisting of: baccatin III and a derivative thereof, paclitaxel and a derivative thereof, taxusin and a derivative thereof, docetaxel and a derivative thereof, and cabazitaxel and a derivative thereof.

Compared with the prior art, the disclosure has the following beneficial effects.

The present disclosure creatively identifies T9αH from *Taxus chinensis,* where the T9αH can catalyze the hydroxylation reaction at the C9 position in the synthesis process of taxane compounds. For example, when synthesizing paclitaxel, the T9αH enzyme can catalyze the conversion of taxadiene-5α,10β,13α-triol into taxadiene-5α,9α,10β,13α-tetraol, thereby achieving a hydroxylation reaction at the C9 position.

Furthermore, the present disclosure co-expresses the above T9αH gene with other identified catalytic enzyme genes in model biological systems such as a plant cell (such as tobacco, etc.) or a microbial cell (such as *Escherichia coli, Saccharomyces cerevisiae,* etc.), thereby ultimately synthesizing the precursor substance Baccatin III *de novo* in the plant cell or the microbial cell.

Furthermore, the present disclosure fills the gaps of the last few enzymatic reactions in the biosynthesis of taxanes (such as paclitaxel) over the past decades, and ultimately resolves the complete biosynthetic pathway of taxanes (such as paclitaxel). This not only provides a new industrial production route for producing paclitaxel or other taxane compounds, but also can efficiently obtain a large amount of paclitaxel and other taxanes by modifying the pathway enzymes and utilizing low-value precursor substances. This not only meets the requirements of green environmental protection, but also can greatly reduce the price of paclitaxel-related drugs, thereby allowing more people to afford the related drugs, and reducing the burden on patients' families.

### BRIEF DESCRIPTION OF THE DRAWINGS

Fig. 1 is an Extracted Ion Chromatogram of taxusin, a product synthesized by a combination of 7 genes (TXS, T5αH, T13αH, T10βH, T9αH1, TAT, and TAX19), with GFP as a control.
Fig. 2 shows the results of characterizing the activity of T9αH in tobacco, particularly, the structural formula represents taxusin, and the bar graph represents the activity comparison between the gene of known synthase, the three T9αH homologous genes (T9αH1/2/3) and the control (GFP), i.e., comparison of the content of taxusin generated by different hydroxylases, represented by ion abundance.
Fig. 3 is an Extracted Ion Chromatogram of baccatin III, a product synthesized by a combination of 9 genes (TXS, T5αH, T13αH, T9αH1, T2αH, T7βH, TOT, TAT, and TBT), with GFP as a control.
Fig. 4 shows that tobacco can synthesize baccatin III de novo with the participation of T9αH; particularly, the structural formula represents baccatin III, and the bar graph represents the activity comparison between the gene of known synthase, the three T9αH homologous genes (T9αH1/2/3) and the control (GFP), i.e., comparison of the content of baccatin III generated by different hydroxylases, represented by ion abundance.

### DETAILED DESCRIPTION OF THE EMBODIMENTS

The present disclosure discloses a bioenzyme, a nucleic acid molecule, a biological material and disclosures thereof for catalyzing the hydroxylation of C9 position in a taxane compound. Those skilled in the art can refer to the content of this disclosure and appropriately improve the process parameters to achieve it. It should be particularly noted that, all similar substitutions and modifications are obvious to those skilled in the art, and they are deemed to be included in the disclosure. The method and use according to the disclosure have been described through preferred embodiments. Relevant personnel can obviously modify or appropriately modify and combine the method and use described herein without departing from the content, spirit and scope of the disclosure to implement and apply the technology of the disclosure.

### Terminology Explanation:

In the disclosure, the term "bioenzyme" or "enzyme" refers to a protein (or "polypeptide" or "peptide composition") with biological activity, including both naturally occurring proteins and variants and modified forms thereof. As used herein, the terms "protein" and "polypeptide" are used interchangeably, and thus the term polypeptide can be used to refer to a full-length polypeptide as well as a fragment of a full-length polypeptide. The term "fragment" refers to a part of a polypeptide sequence. A "fragment" or "biologically active portion" includes a polypeptide including a sufficient number of contiguous amino acid residues to retain biological activity, such as a polypeptide in which the N-terminal amino acid has been truncated.

"Variant" means a substantially similar sequence. As will be readily appreciated by those skilled in the art, naturally occurring proteins may have some differences between different species or between different samples, which may be deletion and/or addition of one or more amino acids at one or more internal sites and/or substitution of one or more amino acids at one or more sites of the native polypeptide. However, this does not affect their ability to play the same or similar roles, and these proteins can be referred to as natural variants of the exemplary proteins. Modifications of proteins include, but are not limited to, appropriate amino acid substitutions/additions/deletions, truncation of N-terminal amino acid, codon optimization suitable for host cell preferences, tag addition and fusion, etc., which do not affect the biological activity of the target protein. These proteins may be referred to as artificial variants of the reference proteins. Guidance for appropriate amino acid substitutions that do not affect the biological activity of the target protein can refer to the model described in Dayhoff et al. (1978) Atlas of Protein Sequence and Structure (Natl. Biomed. Res. Found., Washington,D.C.), which is incorporated herein by reference. Conservative substitutions, such as replacing one amino acid with another having similar properties, may be optimal.

In the disclosure, the term "amino acid" refers to any amino acid (both standard and non-standard amino acids), including but not limited to: α-amino acid, β-amino acid, γ-amino acid and δ-amino acid. Examples of suitable amino acids include, but are not limited to, alanine, asparagine, aspartic acid, cysteine, glutamic acid, glutamine, glycine, proline, serine, tyrosine, arginine, histidine, isoleucine, leucine, lysine, methionine, phenylalanine, threonine, tryptophan, and valine.

In the present disclosure, the term "taxane 9α hydroxylase" refers to an enzyme that catalyzes hydroxylation of C9 position of the taxane ring skeleton. According to the experimental results of the present disclosure, it may also have the function of catalyzing carbonylation of C9 position of the taxane ring skeleton. In the present disclosure, the term "T9αH" may refer to taxane 9α hydroxylase, or a nucleotide molecule or sequence encoding taxane 9α hydroxylase, and the specific meaning may be determined in combination with the context.

In some embodiments, the bioenzymes related to the disclosure can be isolated from a material including a given bioenzyme from any source. Any method of obtaining the bioenzymes related to the disclosure is compatible with the disclosure.

As used herein, the term "isolating" means removing from its natural environment or from other compound present when the compound is first formed. The term "isolated" includes a material separated from a natural source, as well as a material recovered following production by recombinant expression in a host cell (such as nucleic acids and proteins), or a chemically synthesized compound (such as a nucleic acid molecule, protein and peptide).

In the disclosure, the term "nucleic acid molecule" (or "nucleic acid" or "polynucleotide") may refer to a polymeric form of nucleotides, which may include sense and antisense strands of RNA, cDNA, genomic DNA, as well as synthetic forms and mixed polymers thereof. A nucleotide may refer to ribonucleotide, deoxyribonucleotide, or modified form of either type of nucleotide. As used herein, "nucleic acid molecule" is synonymous with "nucleic acid" and "polynucleotide." A nucleic acid molecule is usually at least 10 bases in length unless otherwise specified. The term can refer to an RNA or DNA molecule of indeterminate length. The term includes both single-stranded and double-stranded forms of DNA. Nucleic acid molecules can include one or both of naturally occurring and modified nucleotides linked together by naturally occurring and/or non-naturally occurring nucleotide linkages.

As will be readily appreciated by those skilled in the art, for nucleotide sequences, well-known molecular biology techniques, such as the polymerase chain reaction (PCR) and hybridization techniques outlined herein, can be used to identify naturally occurring variants, i.e., substantially similar sequences.

As will be readily appreciated by those skilled in the art, nucleic acid molecules may be chemically or biochemically modified, or may include non-natural or derived nucleotide bases. Such modifications include, for example, labeling, methylation, substitution of one or more naturally occurring nucleotides with an analog, internucleotide modifications (e.g., uncharged bonds: e.g., methylphosphonate, phosphotriester, phosphoramidite, carbamate, etc.; charged bonds: e.g., phosphorothioate, phosphorodithioate, etc.; pendant moieties: e.g., peptide; intercalator: e.g., acridine, psoralen, etc.; chelating agents; alkylating agents; and modified bonds: e.g., α-anomeric nucleic acids, etc.). The term "nucleic acid molecule" further includes any topological conformation, including single-stranded, double-stranded, partially double-stranded, triple-stranded, hairpin, circular, and padlocked conformations.

In some embodiments, the nucleic acid molecules related to the disclosure may be cloned from DNA including a given nucleic acid molecule from any source, for example, by PCR amplification and/or restriction enzyme digestion. In some embodiments, the nucleic acid molecules related to this disclosure are synthetic. Any method of obtaining nucleic acid molecules related to the invention is compatible with the disclosure.

In the disclosure, the term "identity" refers to sequence similarity with natural nucleic acid sequences or amino acid sequences. Identity can be assessed by the naked eye or by computer software. Using computer software, the identity between two or more sequences can be expressed as a percentage (%), which can be used to evaluate the identity between related sequences.

Unless otherwise indicated, the sequence identity values provided herein refer to the values obtained using the full-length sequences of the disclosure by Jalview version 2.11.2.7 (Waterhouse, A.M., Procter, J.B., Martin, D.M.A, Clamp, M. and Barton, G.J. (2009) "Jalview Version 2 -- a multiple sequence alignment editor and analysis workbench" Bioinformatics 25(9) 1189-1191 doi:10.1093/bioinformatics/btp033), and using the default parameters in the multiple alignment software package MUSCLE version v3.8.31 ("MUSCLE: multiple sequence alignment with high accuracy and high throughput" Nucleic Acids Res. 32(5):1792 (2004)); or any equivalent program thereof).

In this disclosure, the term "homology" is sometimes used to refer to the level of similarity between two or more nucleic acid sequences or amino acid sequences expressed as a percentage of positional identity (i.e., sequence similarity or identity). Homology also refers to the concept of evolutionary correlation, usually demonstrated by similar functional characteristics between different nucleic acids or proteins that share similar sequences.

As easily understood by technical personnel in this field, homologous sequences can be obtained by aligning known sequences with genome or transcriptome data of species samples with similar evolutionary relationships. For example, between different species of the same genus or between different plants of the same species, the homologous sequence of the sequence can be obtained by aligning the known sequence in the sample genome or transcriptome data. Those skilled in the art can expect it to have the same or similar function.

Additional mathematical algorithms are known in the art and can be used to align two sequences. See, for example, the algorithms in Karlin and Altschul (1990) Proc. Natl. Acad. Sci. USA 87:2264, such as a modification in Altschul (1993) Proc. Natl. Acad. Sci. USA 90:5873-5877. This algorithm was introduced in the BLAST program of Altschul et al. (1990) J.Mol. Biol. 215:403. BLAST nucleotide searches can be performed with the BLASTN program (nucleotide query for nucleotide sequence searches) to obtain nucleotide sequences homologous to the nucleic acid molecules of the disclosure, or with the BLASTX program (translated nucleotide query for protein sequence searches) to obtain protein sequences homologous to the nucleic acid molecules of the disclosure. BLAST protein searches can be performed with the BLASTP program (protein query for protein sequence searches) to obtain amino acid sequences homologous to protein molecules of the disclosure, or with the TBLASTN program (protein query for translated nucleotide sequence searches) to obtain nucleotide sequences homologous to protein molecules of the disclosure. To obtain gapped alignments for comparison purposes, Gapped BLAST (in BLAST 2.0) can be used as described in Altschul et al. (1997) Nucleic Acids Res. 25:3389. Alternatively, PSI-Blast can be used to perform an iterated search that detects distant relationships between molecules. See Altschul et al. (1997) supra. When utilizing BLAST, Gapped BLAST, and PSI-Blast programs, the default parameters of the respective programs (e.g., BLASTX and BLASTN) can be used. Alignment can also be performed manually by inspection.

In this disclosure, the term "synthetic" refers to polynucleotide (i.e., DNA or RNA) molecules produced by chemical synthesis as an in vitro process. For example, synthetic DNA can be produced in a reaction process within an Eppendorf^{™} tube, such that synthetic DNA is enzymatically produced from a natural DNA or RNA strand. Other laboratory methods may be utilized to synthesize polynucleotide sequences. Oligonucleotides can be chemically synthesized on an oligonucleotide synthesizer by solid phase synthesis using phosphoramidites. Synthetic oligonucleotides can anneal to each other as a complex, thereby producing a "synthetic" polynucleotide. Other methods for chemically synthesizing polynucleotides are known in the art, and can be readily adapted for use with the present disclosure.

In this disclosure, the term "gene" refers to a nucleic acid fragment expressing a specific protein. A "gene" includes DNA regions that encode a gene product, as well as all DNA regions that regulate the production of a gene product, whether or not such regulatory sequences are adjacent to the coding and/or transcribed sequences. Thus, genes include, but are not necessarily limited to: promoter sequences, terminators, translation regulatory sequences, such as ribosome binding sites and internal ribosome entry sites, enhancers, silencers, insulators, boundary elements, replication origins, matrix attachment sites, introns, and locus control regions.

In the disclosure, the term "gene product" refers to any product generated by a gene. For example, a gene product can be a direct transcription product of the gene (such as mRNA, tRNA, rRNA, antisense RNA, interfering RNA, ribozyme, structural RNA, or any other type of RNA), or it can be a protein produced by translation of mRNA.

In the disclosure, the term "expression cassette" refers to a DNA fragment that can be inserted into a nucleic acid or polynucleotide at a specific restriction site or by homologous recombination. As used herein, a DNA fragment includes a polynucleotide encoding a polypeptide of interest, and an expression cassette and restriction sites are designed to ensure insertion of the expression cassette into the proper reading frame for transcription and translation. In one embodiment, an expression cassette may include a polynucleotide encoding a polypeptide of interest and, in addition to the polynucleotide, have elements that facilitate transformation of a particular host cell. In one embodiment, the expression cassette may further include elements that allow for enhanced expression of the polynucleotide encoding the polypeptide of interest in the host cell. These elements may include, but are not limited to, promoters, minimal promoters, enhancers, response elements, terminator sequences, polyadenylation sequences, and the like.

In the disclosure, the term "vector" is used interchangeably with "construct", "cloning vector" and "expression vector", and means a vector that can introduce DNA or RNA sequences (such as foreign genes) into host cells to transform the host and promote the expression (such as transcription and translation) of the introduced sequences. "Non-viral vector" refers to any vector that does not include a virus or retrovirus. In some embodiments, a "vector" is a DNA sequence including at least one DNA replication origin and at least one selectable marker gene. Examples include, but are not limited to, plasmids, cosmids, bacteriophages, bacterial artificial chromosomes (BACs), or viruses that carry foreign DNA into cells. The vector may further include one or more genes, antisense molecules and/or selectable marker genes and other genetic elements known in the art. The vector may transduce, transform or infect cells, thereby causing the cells to express the nucleic acid molecules and/or proteins encoded by the vector.

In the disclosure, the term "expression" refers to the biosynthesis of a gene product, including the transcription and/or translation of the gene product. "Expressing" or "producing" a protein or polypeptide from a DNA molecule refers to transcribing and translating the coding sequence to produce the protein or polypeptide, while "expressing" or "producing" a protein or polypeptide from an RNA molecule refers to translating the RNA coding sequence to produce the protein or polypeptide.

Expression vectors including all necessary expression elements are commercially available, and familiar to those skilled in the art. See, e.g., Sambrook et al., Molecular Cloning: A Laboratory Manual, 2nd ed., Cold Spring Harbor Laboratory Press, 1989. Cells are genetically engineered by introducing exogenous DNA (RNA) into the cells. The exogenous DNA (RNA) is placed under the effective control of transcription elements to allow the exogenous DNA to be expressed in the host cell.

In the disclosure, the term "transformation" includes all techniques by which a nucleic acid molecule can be introduced into such a cell. Examples include, but are not limited to: transfection with viral vectors; transformation with plasmid vectors; electroporation; fat infection; microinjection; *Agrobacterium-mediated* transfer; direct DNA uptake; WHISKERS^{™} mediated transformation; and microprojectile bombardment. These techniques can be used for both stable and transient transformation of host cells. "Stable transformation" refers to the introduction of a nucleic acid fragment into the genome of a host organism, resulting in genetic stability. Once stably transformed, the nucleic acid fragment is stably integrated into the genome of the host organism and any subsequent generations. Host organisms including the transformed nucleic acid fragments are referred to as "transgenic" organisms. "Transient transformation" refers to the introduction of a nucleic acid fragment into the nucleus or DNA-including organelle of a host organism, resulting in gene expression without genetically stable inheritance.

In some embodiments, to transform hosts and host cells, the nucleotide sequence of the disclosure can be inserted into any vector known in the art suitable for expressing nucleotide sequences in hosts or host cells by using standard techniques. The choice of vector depends upon the preferred transformation technique and the target host species to be transformed. The transformation method depends on the host cell to be transformed, the stability of the vector used, the expression level of the gene product and other parameters.

In the disclosure, the "stringent conditions" mentioned above may be any one of: low stringency conditions, medium stringency conditions, and high stringency conditions. "Low stringency conditions" include, for example, the conditions of: 5×SSC, 5×Denhardt's solution, 0.5% SDS, 50% formamide, and 32°C. "Moderately stringent conditions" include, for example, the conditions of: 5×SSC, 5×Denhardt's solution, 0.5% SDS, 50% formamide, and 42°C. "High stringent conditions" include, for example, the conditions of: 5×SSC, 5×Denhardt's solution, 0.5% SDS, 50% formamide, and 50°C. Among the above conditions, the higher the temperature, the more efficiently a DNA having high homology can be expected to be obtained. Factors that affect hybridization stringency include: temperature, probe concentration, probe length, ionic strength, time, salt concentration, and other factors. Those skilled in the art can achieve the same stringent conditions by appropriately selecting these factors.

**Table 1: Names of the 9 genes and their NCBI sequence numbers**

| Abbreviation of the name | Full Name | SEQ ID NO: of the gene /NCBI number |
|---|---|---|
| T9αH(or T9H) | Taxane 9α hydroxylase | SEQ ID NO: 15-28 |
| TXS | Taxadiene synthase | U48796 |
| TAT | Taxadien 5-alpha-ol-O-acetyl transferase | AF190130 |
| T5αH | Taxane 5-alpha hydroxylase | AY289209 |
| T13αH | Taxane 13-alpha hydroxylase | AY056019 |
| T2αH | Taxane 2-alpha hydroxylase | AY518383 |
| T7βH | Taxane 7-beta hydroxylase | AY307951 |
| TOT | Taxol oxetanase | SEQ ID NO: 29 |
| TBT | Taxane 2-alpha-O-benzoyl transferase | AF297618 |

| | | |
|---|---|---|
| Note: The sequence numbers of the proteins of the identified genes in Table 1 are all derived from Uniprot. The protein and gene sequences of T9αH and TOT provided in Table 1 are derived from the genus *Taxus.* | | |

**Table 2: Similarity of the amino acid sequences of bioenzymes**

| Name of bioenzyme | SEQ ID NO: | Similarity with T9αH1 | Source |
|---|---|---|---|
| T9αH1(orT9H1) | 1 | 100% | *Taxus chinensis* |
| T9αH2(orT9H2) | 2 | 83.86% | *Taxus chinensis* |
| T9αH3(orT9H3) | 3 | 67.27% | *Taxus chinensis* |
| T9αH4(orT9H4) | 4 | 99.60% | *Taxus chinensis* |
| T9αH5(orT9H5) | 5 | 67.47% | *Taxus chinensis* |
| T9αH6(orT9H6) | 6 | 99.60% | *Taxus cuspidate* |
| T9αH7(orT9H7) | 7 | 67.66% | *Taxus cuspidate* |
| T9αH8(orT9H8) | 8 | 99.00% | *Taxus yunnanensis* |
| T9αH9(orT9H9) | 9 | 67.27% | *Taxus yunnanensis* |
| T9αH10(orT9H10) | 10 | 65.67% | *Taxus yunnanensis* |
| T9αH11(orT9H1 1) | 11 | 65.07% | *Taxus yunnanensis* |
| T9αH12(orT9H12) | 12 | 99.00% | *Taxus baccata* |
| T9αH13(orT9H13) | 13 | 63.75% | *Taxus baccata* |
| T9αH14(orT9H14) | 14 | 63.15% | *Taxus baccata* |

**Table 3: Nucleotide sequence similarity of bioenzymes**

| Name of Gene | SEQ ID NO: | Similarity with T9αH1 |
|---|---|---|
| T9αH1(orT9H1) | 15 | 100% |
| T9αH2(orT9H2) | 16 | 88.47% |
| T9αH3(orT9H3) | 17 | 77.54% |
| T9αH4(orT9H4) | 18 | 99.80% |
| T9αH5(orT9H5) | 19 | 77.71 % |
| T9aH6(orT9H6) | 20 | 99.34% |
| T9αH7(orT9H7) | 21 | 77.86% |
| T9αH8(orT9H8) | 22 | 99.34% |
| T9αH9(orT9H9) | 23 | 77.64% |
| T9αH10(orT9H10) | 24 | 75.84% |
| T9αH11(orT9H11) | 25 | 75.83% |
| T9αH12(orT9H12) | 26 | 99.14% |
| T9αH13(orT9H13) | 27 | 75.48% |
| T9αH14(orT9H14) | 28 | 75.83% |

**Table 4 Amino acids and their English names and abbreviations**

| Name | Three-letter abbreviation | One-letter abbreviation |
|---|---|---|
| Glycine | Gly | G |
| Alanine | Ala | A |
| Valine | Val | V |
| Leucine | Leu | L |
| Isoleucine | Ile | I |
| Proline | Pro | P |
| Phenylalanine | Phe | F |
| Tyrosine | Tyr | Y |
| Tryptophan | Trp | W |
| Serine | Ser | S |
| Threonine | Thr | T |
| Cystine | Cys | C |
| Methionine | Met | M |
| Asparagine | Asn | N |
| Glutamine | Gln | Q |
| Asparticacid | Asp | D |
| Glutamicacid | Glu | E |
| Lysine | Lys | K |
| Arginine | Arg | R |
| Histidine | His | H |

The reagents, instruments or biological materials used in the present disclosure are commercially available.

The present disclosure will be further described below in conjunction with the Examples

### Example 1: Expression of Taxane C9 Hydroxylase T9αH Gene in Tobacco

### 1. cDNA Acquisition

*Taxus chinensis* var. *maire* was preserved in the laboratory. After collecting the young leaves, they were immediately quick-frozen in liquid nitrogen. Some samples were taken to grind in a mortar. 100 mg of ground sample was added to a 1.5 mL centrifuge tube, and lysis buffer was added to extract RNA (Plant Total RNA Isolation Kit Plus, FOREGENE, China). The mRNA was reverse transcribed into cDNA by using a reverse transcription kit (HiScript III 1st Strand cDNA Synthesis Kit, Vazyme, China).

### 2. Cloning of Taxane C9 Hydroxylase T9αH

The genome of *Taxus chinensis* var. mairei was analyzed, and the amino acid sequence and nucleotide sequence of the reading frame of T9αH are represented by SEQ ID NO: 1 (its homologous sequences are represented by SEQ ID NOs: 2-14 in the sequence listing) and SEQ ID NO: 15 (its homologous sequences are represented by SEQ ID NOs: 16-28 in the sequence listing), respectively.

The amino acid sequence of the reading frame of T9αH1 (SEQ ID NO: 1):

MDSLSFLKSMEAKFGQVIHRDQSSSTALLSLAFTAALAIFLVLLFRFKSRPSTNFPPGNFGFPFIGETIQFLRAL
RSESPHMFFDERLKKFGRVFKTSLTGHPTAVFCGPAGNRFIYSNEHKLVQSSGPNSFVKLVGQQSIVTKTGEE
HRIFRGVLNEFLGPHALQSYTPKMSSKIQENINKHWKGKDEVNMLPSIRQLVFSISSSLFFDINDEDQQEQLK
TLLETILVGTLSVPLDIPGSNFRKALRARSKLDEILSRLIESRRKDMRSGIASTSKNLLSVLLAFKDERGNPLT
DTEILDNFSFMLHASYDTTVSPTVCIFKLLSANPECYEKVVQEQLGILGNKKDGEEICWNDLKAMKYTWQ
AAQETMRLFPPAFGSFRKVIADIHHDGYIIPKGWKAMVTNYSTSRKEEYFDEPDKFKPSRFGDGKYVAPYTF
LPFGAGIRICPGWEFAKLEMLLFIHHFVKNFSGYLPLDTKEKISGDPFPPLPKNGFPIKLFPRT.

The nucleotide sequence of the reading frame of T9αH1 (SEQ ID NO: 15):

ATGGATTCCTTAAGTTTTCTAAAAAGCATGGAAGCGAAATTCGGCCAAGTCATACACCGGGATCAGTCTT

CCAGTACTGCTCTTCTGTCCCTCGCATTCACAGCTGCTCTTGCCATTTTTCTTGTGTTGCTCTTTCGATTT

AAAAGCCGGCCCTCTACTAATTTCCCTCCAGGAAATTTTGGCTTCCCTTTCATTGGAGAGACGATACAGT

TCTTGCGGGCACTTCGATCAGAATCGCCTCATATGTTTTTTGATGAGAGATTGAAGAAATTTGGGCGTGT

ATTCAAGACGTCATTAACTGGGCATCCCACAGCTGTGTTCTGCGGGCCTGCGGGAAACCGGTTTATTTAC

TCGAATGAGCACAAGCTGGTGCAGTCGTCTGGGCCCAACTCCTTCGTCAAACTGGTTGGGCAGCAATC

CATCGTGACCAAAACAGGAGAGGAGCACCGCATCTTTCGTGGTGTCCTGAACGAGTTTCTGGGGCCTC

ATGCCTTACAGAGTTATACGCCTAAAATGAGTTCCAAAATCCAGGAGAATATCAATAAGCATTGGAAGGG

TAAAGATGAAGTGAACATGCTTCCTTCGATAAGACAGCTCGTCTTCTCCATTTCAAGCAGCTTGTTTTTT

GATATTAATGATGAGGATCAACAGGAACAACTTAAAACTCTTTTAGAAACTATTCTTGTTGGAACTTTGT

CGGTTCCCCTCGACATTCCAGGATCTAATTTTCGTAAAGCTCTTCGGGCGCGTTCCAAGCTGGATGAAAT

TCTGTCTCGTTTAATCGAAAGCAGAAGAAAAGATATGCGTTCTGGGATAGCTTCTACCAGTAAAAATCTA

CTGTCGGTGCTGCTCGCCTTCAAAGATGAAAGAGGGAATCCATTGACGGACACGGAGATCCTCGACAA

CTTTTCTTTTATGCTTCACGCCTCATACGACACCACCGTTTCGCCCACGGTTTGTATATTTAAGCTGCTCT

CCGCCAATCCAGAATGCTATGAAAAAGTAGTTCAAGAACAATTGGGAATACTTGGCAATAAAAAGGACG

GTGAAGAAATCTGTTGGAACGATCTGAAAGCTATGAAATATACATGGCAAGCAGCTCAAGAAACAATGA

GGCTTTTCCCTCCAGCGTTTGGATCATTTCGCAAGGTCATCGCCGATATTCATCATGATGGCTATATAATTC

CCAAAGGATGGAAAGCTATGGTGACAAATTACAGTACAAGTAGGAAAGAAGAGTACTTCGATGAACCA

GACAAATTCAAGCCTTCAAGATTTGGGGATGGAAAGTATGTGGCTCCGTACACGTTCTTACCTTTCGGG
GCAGGAATACGCATATGCCCAGGATGGGAGTTCGCTAAGTTGGAGATGTTACTGTTCATCCATCATTTTG
TCAAAAATTTCAGCGGATACCTCCCACTTGACACCAAGGAAAAGATTTCCGGAGATCCATTCCCTCCTC
TCCCCAAAAATGGATTTCCCATTAAACTATTTCCCAGAACCTAA.

Based on the nucleotide sequence of the T9αH1 gene, primers P1 and P2 for amplifying the T9αH1 gene were designed. The primers included a part of the sequence of the tobacco expression vector pEAQ-HT. The T9αH1 gene was amplified by PCR using the cDNA of *Taxus chinensis var. mairei* leaves as a template. The PCR target product was recovered by gel excision, and then recombined with the linear pEAQ-HT vector digested with RruI and XhoI. The ClonExpress one-step cloning kit (Vazyme Biotech) was used for cloning and sequencing, and the positive recombinant plasmid was named pEAQ-HT- T9αH1.

Using the same method, pEAQ-HT-T9αH2 and pEAQ-HT-T9αH3 were obtained.

Among the primers used, P1 and P2 were used to amplify T9αH1, P3 and P4 were used to amplify T9αH2, and P5 and P6 were used to amplify T9αH3. The primer sequences are shown in Table 5.

**Table 5: Primer sequences**

| Name | SEQ ID No: | Sequence |
|---|---|---|
| P1 | 30 | 5'-gtatattctgcccaaattcgcgaATGGATTCCTTAAGTTTTCTAAAAAGCATG-3' |
| P2 | 31 | 5'-tgaaaccagagttaaaggcctcgagTTAGGTTCTGGGAAATAGTTTAATGGGAAATC-3' |
| P3 | 32 | 5'- gtatattctgcccaaattcgcgaATGGATTGCTTCAATTTTCTAACAAGC-3' |
| P4 | 33 | 5'-tgaaaccagagttaaaggcctcgagTTAGGTTCTGGGAAGGAGTTTTATGG-3' |
| P5 | 34 | 5'-gtatattctgcccaaattcgcgaATGGATCGCTTCAATTTGCTG-3' |
| P6 | 35 | 5'-tgaaaccagagttaaaggcctcgagCTAGGATCTGGGAAAAAGTTTCATAGAAAAT-3' |

| | | |
|---|---|---|
| Note: The lowercase sequence is the vector sequence, and the uppercase sequence is the T9αH specific primer sequence. | | |

### 3. Functional verification of the taxane C9 hydroxylase T9αH gene in tobacco

The constructed expression vector pEAQ-HT-T9αH was transferred into *Agrobacterium* GV3101 to obtain the GV3101/pEAQ-HT-T9αH transgenic strain; a positive single clone was picked to inoculate into LB medium (including 50 µg/mL kanamycin and 25 µg/mL rifampicin) to culture at 28°C for 24 h; the cultured bacterial solution was taken to add in 10 mL LB medium (including 50 µg/mL kanamycin and 25 µg/mL rifampicin) at a ratio of 1:100 to culture at 28°C overnight to an OD₆₀₀ value of 0.8-1.0; the resuspension MMA (10 mM MES, 10 mM MgCl₂, 150 µM acetosyringone) was added to a final OD₆₀₀ of 0.8-1.0, allowing to stand at room temperature for 1-2 h.

The *resuspended Agrobacterium* combination (known gene + T9αH homologous gene or GFP) was injected into the back of leaves of 4-6 week-old *Nicotiana benthamiana* by using a 1 mL syringe without the needle. After drying under light for 1-2 hours, the leaves were transferred to a dark place for incubation for 24h, and then transferring to normal light for incubation. Five days after the injection *of Agrobacterium,* the samples were collected to inject into the leaves with GFP as controls. The above known genes include TXS (U48796), T5αH (AY289209), T13αH (AY056019), TAX19 (AY628434), TAT (AF190130), and T10βH (AF318211).

The tobacco sample was ground with a grinder, and the sample powder was transferred to a 5 mL centrifuge tube. After freeze-drying, 1 mL of methanol was added to perform ultrasonic extraction for 30 min. After centrifugation at 13,000 rpm for 15 min, the supernatant was transferred to a new centrifuge tube to centrifuge at 13,000 rpm for 15 min. 200 µL was taken out to put into a sample injection bottle for detection in LC-MS. By comparing with the taxusin standard, it was found that a substance with the same retention time and molecular weight as taxusin could be detected from tobacco.

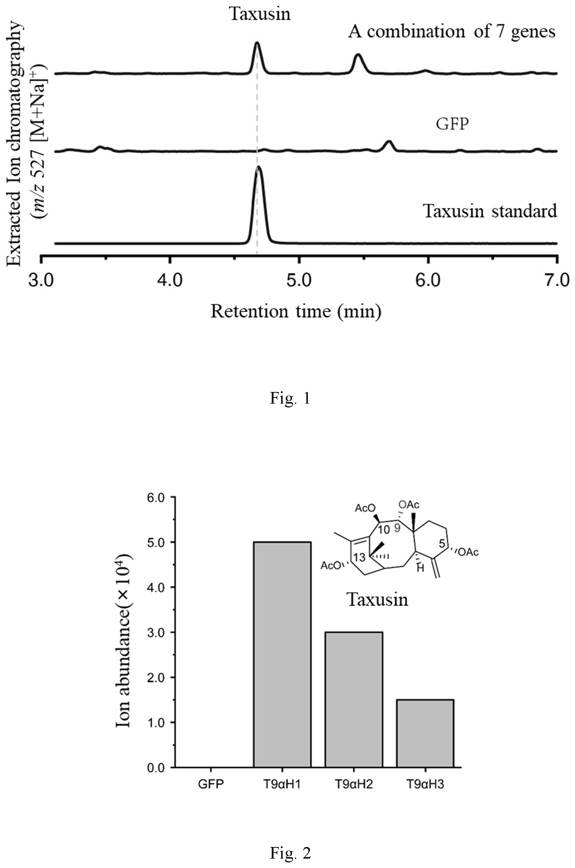

As shown in Figs. 1 and 2, in the combination including T9αH, taxusin can be synthesized *de novo* in tobacco leaves, and the activities of its homologous genes have certain differences.

### Example 2: Taxane C9 Hydroxylase T9αH gene is Involved in the Synthesis of Baccatin III

### 1. Taxane C9 hydroxylase T9αH gene is involved in the de novo synthesis of baccatin III in tobacco

The constructed expression vector pEAQ-HT-T9αH was transferred into *Agrobacterium* GV3101 to obtain the GV3101/pEAQ-HT-T9αH transgenic strain. The positive single clone was picked and inoculated into LB medium (including 50 µg/mL kanamycin and 25 µg/mL rifampicin), culturing at 28°C for 24 h. The cultured bacterial liquid was taken in 10 mL LB medium (including 50 µg/mL kanamycin and 25 µg/mL rifampicin) at a ratio of 1:100, culturing at 28°C overnight to an OD₆₀₀ value of 0.8-1.0; adding resuspension MMA (10 mM MES, 10 mM MgCl₂, 150 µM acetosyringone) to the final OD₆₀₀ of 0.8-1.0, and allowing to stand at room temperature for 1-2 h.

In the same manner, CV3101 *Agrobacterium* including genes such as TXS (U48796), TAT (AF190130), T5αH (AY289209), T13αH (AY056019), T2αH (AY518383), T7βH (AY307951), TOT (SEQ ID NO: 29), and TBT (AF297618) were respectively cultured, and resuspended in MMA to an OD₆₀₀ value of 0.8-1.0, and mixing with *Agrobacterium* including T9αH at a ratio of 1:1, *and Agrobacterium* including the fluorescent protein GFP was used as a control.

The resuspended *Agrobacterium* combination was injected into the back of 4-6 week-old *Nicotiana benthamiana* leaves by using a 1 mL syringe without a needle. After drying under light for 1-2 h, the leaves were transferred to a dark place for incubation for 24 h, and then transferring to normal light for incubation. Five days after the injection *of Agrobacterium,* the samples were collected to inject into the leaves with GFP as controls.

The tobacco sample was ground with a grinder, and the sample powder was transferred to a 5 mL centrifuge tube. After freeze-drying, 1 mL of methanol was added to perform ultrasonic extraction for 30 min. After centrifugation at 13,000 rpm for 15 min, the supernatant was transferred to a new centrifuge tube to centrifuge at 13,000 rpm for 15 min. 200 µL was taken out to put into an injection bottle for detection in LC-MS. By comparing with the standard of baccatin III, it was found that a substance with the same retention time and molecular weight as baccatin III could be detected in tobacco.

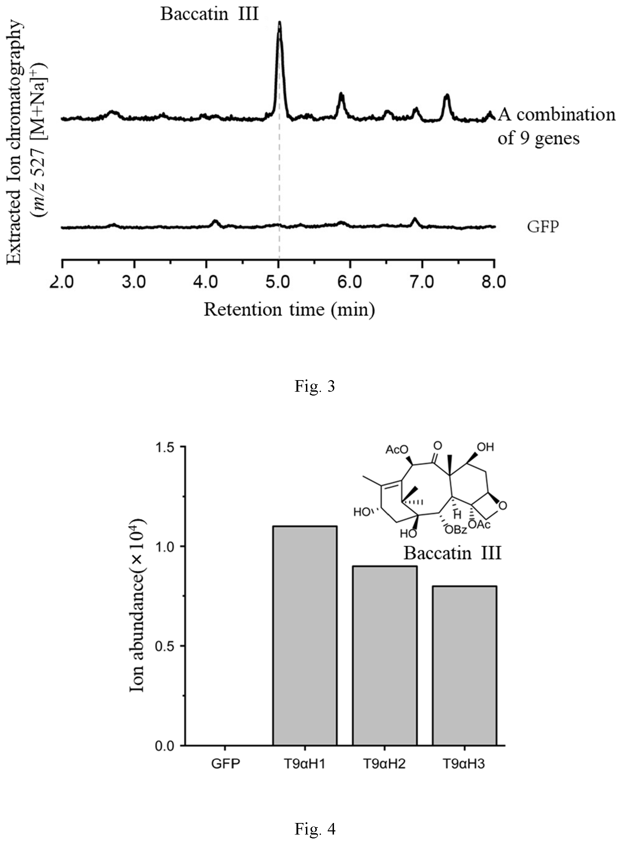

As shown in Figs. 3-4, in the combination including T9αH, baccatin III can be synthesized *de novo* in tobacco leaves, and the activities of its homologous genes have certain differences.

The above described are only preferred embodiments of the disclosure. It should be pointed out that for ordinary technicians in this technical field, several improvements and modifications can be made without departing from the principle of the disclosure. These improvements and modifications should also be regarded as falling in the protection scope of the disclosure.

## Claims

1. A bioenzyme T9αH, **characterized in that** its amino acid sequence comprises at least one of the following sequences:
a1) an amino acid sequence represented by SEQ ID NO: 1;
a2) a fusion amino acid sequence obtained by connecting a tag to N-terminus and/or C-terminus of the amino acid sequence represented by SEQ ID NO: 1;
a3) an amino acid sequence obtained by substitution and/or deletion and/or addition of one or more amino acid residues in the amino acid sequence represented by SEQ ID NO: 1 and having the same function; or
a4) an amino acid sequence having at least 60% identity with the amino acid sequences of SEQ ID NO: 1 and having the same function.

2. The bioenzyme T9αH according to claim 1, **characterized in that**, in a4), the amino acid sequence having at least 60% identity with the amino acid sequence represented by SEQ ID NO: 1 and having the same function comprises at least one of the amino acid sequences represented by SEQ ID NOs: 2-14.

3. A nucleic acid molecule encoding the bioenzyme according to claim 1, **characterized in that** its nucleotide sequence comprises at least one of the following sequences:
b1) a nucleotide sequence represented by SEQ ID NO: 15;
b2) a complementary sequence, degenerate sequence or homologous sequence of the nucleotide sequence represented by SEQ ID NO: 15, wherein the homologous sequence is a nucleotide sequence having at least 75% identity with the nucleotide sequence represented by SEQ ID NO: 15; or
b3) a nucleotide sequence hybridizing with the nucleotide sequence of b1) or b2) under stringent conditions and being capable of encoding a protein having the same function.

4. The nucleic acid molecule according to claim 3, **characterized in that**, in b2), the homologous sequence comprises at least one of the nucleotide sequences represented by SEQ ID NOs: 16-28.

5. A combination of bioenzymes, **characterized by** comprising the bioenzyme T9αH according to claim 1 or 2, and further comprises at least one of: TXS, TAT, T5αH, T13αH, T2αH, T7βH, TOT, or TBT.

6. A biomaterial, **characterized in that** the biomaterial is any one of the following c1) to c3):
c1) an expression cassette comprising the nucleic acid molecule according to claim 3 or 4;
c2) a vector comprising the nucleic acid molecule according to claim 3 or 4, or comprising the expression cassette of c1);
c3) a host cell comprising at least one of: the nucleic acid molecule according to claim 3 or 4, the expression cassette of c1), and the vector of c2);
optionally, the host cell comprises at least one of: microbial cell, plant cell, animal cell, or algae cell.

7. The biomaterial according to claim 6, **characterized in that** the vector comprises pFastBac1, pYES2, pYES2.1, pESC-Ura, pESC-Trp, pESC-Leu, pESC-His, pGEX2T, pTAex3, pUSA, pYMB0, pHT43, pET28b, pIJ702, pUCP19, pEAQ-HT, pYMB03, or pHT43; and/or
the microbial cell comprises at least one of: *Streptomyces, Pseudomonas, Bacillus,* yeast cell, or *Escherichia coli;* and/or
the plant cell comprises at least one of: tobacco cell, *Artemisia caruifolia* cell, *Arabidopsis thaliana* cell, *Physcomitrium* patens cell, *Marchantia polymorpha* cell, tomato cell, ginseng cell, cotton cell, sugarcane cell, potato cell, corn cell, wheat cell, rice cell, radish cell, or lettuce cell; and/or
the animal cell comprises at least one of: insect cell, mammalian cell, fruit fly cell, nematode cell, or fish cell; and/or
the algal cell comprises at least one of: cyanobacteria cell or green algae cell.

8. The biomaterial according to claim 5 or 6, **characterized in that** the host cell further comprises at least one of: TXS, TAT, T5αH, T13αH, T2αH, T7βH, TOT, or TBT.

9. A method for producting a host cell, **characterized by** comprising transforming a host cell with at least one of: the nucleic acid molecule according to claim 3 or 4, the expression cassette or vector in the biomaterial according to any one of claims 6-8; optionally, the method further comprises transforming the host cell with at least one of: TXS, TAT, T5αH, T13αH, T2αH, T7βH, TOT, or TBT.

10. A method for producting a plant or plant cell, **characterized by** comprising transforming the plant or plant cell with at least one of: the nucleic acid molecule according to claim 3 or 4, the expression cassette or vector in the biomaterial according to any one of claims 6-8; optionally, the method further comprises transforming the plant or plant cell with at least one of: TXS, TAT, T5αH, T13αH, T2αH, T7βH, TOT, or TBT.

11. A method for producting the bioenzyme according to claim 1 or 2, **characterized in that** the method comprises transforming a host cell with at least one of: the nucleic acid molecule according to claim 3 or 4, or the expression cassette or vector in the biological material according to any one of claims 6-8, so that the host cell produces the bioenzyme.

12. Use of the bioenzyme according to claim 1 or 2, the nucleic acid molecule according to claim 3 or 4, or the biological material according to any one of claims 6-8, for purpose of the following d1), d2), d3) and/or d4):
d1) catalyzing hydroxylation of C9 position in a precursor substance of a taxane compound and/or an intermediate thereof; a precursor substance of a taxane compound and/or an intermediate thereof is a precursor substance in which the C9 position has not been hydroxylated or oxidized;
d2) synthesizing taxusin and/or an intermediate thereof;
d3) synthesizing baccatin III and/or an intermediate thereof; and
d4) synthesizing a taxane compound and/or an intermediate thereof.

13. The use according to claim 12, **characterized in that** the taxane compound comprises at least one of: paclitaxel and a derivative thereof, taxusin and a derivative thereof, docetaxel and a derivative thereof, or cabazitaxel and a derivative thereof; and/or
the intermediate of the taxane compound comprises at least one of: taxadiene-hexaol-hexaacetate and a derivative thereof, 1-dehydroxybaccatin IV and a derivative thereof, baccatin III and a derivative thereof, 10-deacetylbaccatin III and a derivative thereof, paclitaxel and a derivative thereof, baccatin IV and a derivative thereof, baccatin VI and a derivative thereof, baccatin VII and a derivative thereof, baccatin IX and a derivative thereof, baccatin V and a derivative thereof, taxusin and a derivative thereof, baccatin I and a derivative thereof, taxadiene-2α,5α,7β,9β,10β,13α-hexaol-5α-acetate and a derivative thereof, 5α-acetate-taxuspine F and a derivative thereof, taxadiene-hexaol-tetraacetate and a derivative thereof, or taxuspine F and a derivative thereof.

14. A method for synthesizing a taxane compound and/or an intermediate thereof in vivo or in vitro, **characterized by** comprising utilizing at least one of the precursor substance of a taxane compound and/or an intermediate thereof as a substrate to hydroxylate the C9 position under the catalytic action of the bioenzyme according to claim 1 or 2 and/or the bioenzyme produced by the method according to claim 11; and wherein the precursor substance of the taxane compound and/or an intermediate thereof is a precursor substance in which the C9 position has not been hydroxylated and oxidized.

15. The method according to claim 14, **characterized in that** the precursor substance of the taxane compound and/or an intermediate thereof comprises a compound represented by following Formula I and/or Formula II: 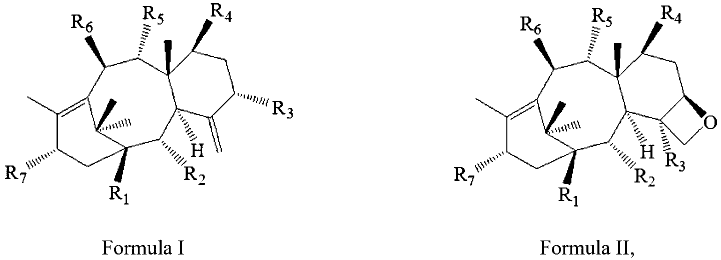 wherein R₁, R₃ and R₄ are each independently selected from: H, -OH and -OAc; R₂ and R₆ are each independently selected from: H, -OH, -OAc and -OBz; R₅ and R₇ are each independently selected from: H, -OH, -OAc and =O.

16. The method according to claim 14 or 15, **characterized in that** the precursor substance of the taxane compound and/or an intermediate thereof comprises at least one of: taxa-4(5),11(12)-diene, taxa-4(20),11(12)-diene-5α- ol, taxadiene-5α,10β,13α-triol, and/or a derivative thereof.

17. The method according to any one of claims 14-16, **characterized in that** the bioenzyme for synthesizing a taxane compound and/or an intermediate thereof further comprises at least one of: TXS, TAT, T5αH, T13αH, T2αH, T7βH, TOT, TBT, T10βH, T14βH, DBAT, BAPT, T2'OH, DBTNBT, PAM, CoA ligase, TAX9, TAX14 or TAX19.

18. The method according to any one of claims 14-17, **characterized in that** the in vivo comprises in microbial cell, algae cell, plant cell and/or animal cell.

19. The method according to any one of claims 14-18, **characterized in that** the in vivo synthesis of a taxane compound and/or an intermediate thereof comprises: allowing the nucleic acid molecule of claim 3 or 4 to express a bioenzyme in microbial cell, algal cell, plant cell, and/or animal cell; and then utilizing the precursor substance of the taxane compound and/or an intermediate thereof as a substrate to synthesize the taxane compound and/or an intermediate thereof under the catalytic action of the bioenzyme.

20. A method for synthesizing baccatin III, **characterized in that** geranylgeranylpyrophosphate (GGPP) is used as a substrate to synthesize baccatin III under the action of the bioenzymes of T9αH, TXS, TAT, T5αH, T13αH, T2αH, T7βH, TOT, and TB; preferably the method comprises synthesizing baccatin III by using the host cell according to claim 6, wherein the host cell comprises heterologous T9αH, TXS, TAT, T5αH, T13αH, T2αH, T7βH, TOT, and TB.

21. A plant or a plant part thereof, **characterized in that** the plant is one of the following plants:
e1) a transgenic plant comprising the nucleic acid molecule according to claim 3 or 4;
e2) a plant formed by growth of the plant cell according to any one of claims 6-8;
e3) a plant produced by the method according to claim 9;
e4) an offspring formed by self-pollination of any one of the plants of e1) to e3), as well as a plant formed by growth of the offspring; and
e5) an offspring formed by hybridization of any one of the plants of e1) to e3) with other varieties, as well as a plant formed by growth of the offspring;
preferably the plant part is root, stem, leaf, flower, fruit, pollen, or seed.

22. A method of manufacturing a commercial product, **characterized in that** the method comprises: obtaining the plant or plant part thereof according to claim 21, and manufacturing the commercial product from the plant or plant part thereof;
preferably the commercial product is selected from the group consisting of: a crude extract, an active pharmaceutical ingredient, and/or a pharmaceutical formulation comprising at least one of baccatin III and a derivative thereof, paclitaxel and a derivative thereof, taxusin and a derivative thereof, docetaxel and a derivative thereof, or cabazitaxel and a derivative thereof.
